(19) Europäisches Patentamt / European Patent Office / Office européen des brevets

(11) **EP 2 737 903 B1**

(12) **EUROPEAN PATENT SPECIFICATION**

(45) Date of publication and mention
of the grant of the patent:
**20.02.2019 Bulletin 2019/08**

(21) Application number: **11870403.0**

(22) Date of filing: **29.07.2011**

(51) Int Cl.:
*A23L 2/52* *(2006.01)*     *A61K 38/43* *(2006.01)*
*A61P 31/04* *(2006.01)*     *A61P 43/00* *(2006.01)*
*C12N 9/22* *(2006.01)*      *A23L 33/19* *(2016.01)*
*A23K 20/189* *(2016.01)*    *A23K 20/147* *(2016.01)*
*A01N 63/00* *(2006.01)*     *A61K 38/17* *(2006.01)*

(86) International application number:
**PCT/JP2011/067491**

(87) International publication number:
**WO 2013/018169 (07.02.2013 Gazette 2013/06)**

(54) **ANTIMICROBIAL ACTIVITY ENHANCING AGENT**

MITTEL ZUR VERSTÄRKUNG EINER ANTIMIKROBIELLEN WIRKUNG

AMPLIFICATEUR DE L'ACTIVITÉ ANTIMICROBIENNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.06.2014 Bulletin 2014/23**

(73) Proprietor: **Morinaga Milk Industry Co., Ltd.
Minato-ku
Tokyo 108-8384 (JP)**

(72) Inventors:
• **MURATA, Mai**
**Zama-shi,**
**Kanagawa 252-8583 (JP)**
• **WAKABAYASHI, Hiroyuki**
**Zama-shi,**
**Kanagawa 252-8583 (JP)**
• **YAMAUCHI, Koji**
**Zama-shi,**
**Kanagawa 252-8583 (JP)**

(74) Representative: **Vossius & Partner
Patentanwälte Rechtsanwälte mbB
Siebertstrasse 3
81675 München (DE)**

(56) References cited:
**WO-A2-2007/142542     WO-A2-2009/118771
JP-A- 6 312 922     JP-A- 7 048 274
JP-A- 2006 006 257     JP-A- 2006 187 300
US-A1- 2008 255 340**

• **PAUL HARRIS ET AL: "Characterisation of the
anti-microbial activity of bovine milk
ribonuclease4 and ribonuclease5 (angiogenin)",
INTERNATIONAL DAIRY JOURNAL, vol. 20, no.
6, 1 June 2010 (2010-06-01), pages 400-407,
XP055075177, ISSN: 0958-6946, DOI:
10.1016/j.idairyj.2009.12.018**
• **HARRIS P. ET AL.: 'Characterisation of the
anti-microbial activity of bovine milk
ribonuclease4 and ribonuclease5 (angiogenin)'
INTERNATIONAL DAIRY JOURNAL vol. 20, no. 6,
June 2010, pages 400 - 407, XP055075177**

EP 2 737 903 B1

SEQUENCE LISTING

<110> Morinaga Milk Industry Co., Ltd.

<120> Enhancer of anti-microorganism activity

<130> FP226-11183

<160> 4

<170> PatentIn version 3.5

<210> 1
<211> 36
<212> PRT
<213> Bos taurus

<400> 1

```
        Val Ser Gln Pro Glu Ala Thr Lys Cys Phe Gln Trp Gln Arg Asn Met
        1               5                   10                  15

        Arg Lys Val Arg Gly Pro Pro Val Ser Cys Ile Lys Arg Asp Ser Pro
                        20                  25                  30
        Ile Gln Cys Ile
                        35
```

<210> 2
<211> 11
<212> PRT
<213> Bos taurus

<400> 2
G1y Arg Arg Arg Arg Ser Val Gln Trp Cys Ala
1 5 10

<210> 3
<211> 25
<212> PRT
<213> Bos taurus

<400> 3

```
Phe Lys Cys Arg Arg Trp Gln Trp Arg Met Lys Lys Leu Gly Ala Pro
1               5               10                  15
Ser Ile Thr Cys Val Arg Arg Ala Phe
            20                  25
```

<210> 4
<211> 147
<212> PRT
<213> Bos taurus

<400> 4

```
Met Ala Leu Gln Arg Thr Gln Ala Phe Leu Leu Leu Leu Leu Leu Thr
1               5               10                  15
Leu Leu Gly Leu Gly Leu Val Gln Pro Ser Tyr Gly Gln Asp Arg Met
            20                  25                  30
Tyr Gln Arg Phe Leu Arg Gln His Val Asp Pro Asp Glu Thr Gly Gly
            35                  40                  45
Asn Asp Ser Tyr Cys Asn Leu Met Met Gln Arg Arg Lys Met Thr Ser
            50                  55                  60

His Gln Cys Lys Arg Phe Asn Thr Phe Ile His Glu Asp Leu Trp Asn
65                  70                  75                  80
Ile Arg Ser Ile Cys Ser Thr Thr Asn Ile Gln Cys Lys Asn Gly Gln
                85                  90                  95
Met Asn Cys His Glu Gly Val Val Arg Val Thr Asp Cys Arg Glu Thr
            100                 105                 110
Gly Ser Ser Arg Ala Pro Asn Cys Arg Tyr Arg Ala Lys Ala Ser Thr
            115                 120                 125
Arg Arg Val Val Ile Ala Cys Glu Gly Asn Pro Glu Val Pro Val His
            130                 135                 140
Phe Asp Lys
145
```

**Description**

Technical Field

The present invention relates to an antimicrobial activity enhancing agent for a lactoferrin hydrolysate containing ribonuclease-4 as an active ingredient.

Background Art

Lactoferrin (LF) is an iron-binding protein contained in tear, saliva, peripheral blood, milk and so forth in the living body, and it is known to show an antimicrobial activity against harmful microorganisms, such as *Escherichia coli, Candida* yeasts and *Clostridium* bacteria (Non-patent document 1). Further, it is also known to show an antimicrobial activity against *Staphylococcus* and Enterococcus bacteria at a concentration of 0.5 to 30 mg/ml (Non-patent document 2).

Further, it is also known a composition containing a lactoferrin hydrolysate for inhibiting adhesion of enteropathogenic *Escherichia coli* to alimentary canal epithelial cells (Patent document 1).

In addition to the above, it is known that Lactoferricin (registered trademark), which is a lactoferrin-derived peptide obtainable from a pepsin digestion product of lactoferrin, has an antimicrobial activity (Non-patent documents 3 and 4).

Furthermore, it is investigated a method for enhancing antimicrobial activity of lactoferrin or lactoferrin hydrolysate mentioned above by combining an ingredient other than such lactoferrins.

For example, it is known an antimicrobial agent containing a compound selected from the group consisting of decomposition products of lactoferrins, lactoferrin-related antimicrobial peptides, and arbitrary mixtures of these, and a casein decomposition product as active ingredients (Patent document 2).

Further, it is also known an antimicrobial agent containing a compound selected from the group consisting of decomposition products of lactoferrins, lactoferrin-related antimicrobial peptides, and arbitrary mixtures of these, and a metal-binding protein as active ingredients (Patent document 3). As the metal-binding protein, lactoferrins, transferrin, conalbumin and casein phosphopeptides are mentioned.

Further, it is disclosed an antimicrobial agent containing a decomposition product of lactoferrins and/or an antimicrobial peptide derived from the decomposition product, and lysozyme as active ingredients (Patent document 4).

Further, it is known a composition consisting of an emulsion of an enzymatic decomposition product of lactoferrins, an antimicrobial peptide obtainable from an enzymatic decomposition product of lactoferrins or a chemically synthesized peptide containing a part of amino acid sequence of lactoferrins, and an aliphatic acid (Patent document 5).

In addition, it is known an antimicrobial agent comprising a newquinolone antimicrobial agent and a hydrolysate of lactoferrins or an antimicrobial peptide derived from a hydrolysate of lactoferrins as active ingredients (Patent document 6).

Compositions comprising lactoferrin and angiogenin and their antimicrobial potential are also known (patent document 7 and 8).

Prior art references

Patent documents

Patent document 1: Japanese Patent Laid-open No. 11-292789
Patent document 2: Japanese Patent Laid-open No. 5-255109
Patent document 3: Japanese Patent Laid-open No. 5-320067
Patent document 4: Japanese Patent Laid-open No. 6-199698
Patent document 5: Japanese Patent Laid-open No. 9-124504
Patent document 6: Japanese Patent Laid-open No. 11-92375
Patent document 7: US2008/255340
Patent document 8: WO2007/142542

Non-patent documents

Non-patent document 1: Welsh, J.K. et al., Journal of Pediatrics, Vol. 94, pages 1-9, 1979

Non-patent document 2: Nonnecke, B.J. et al., Journal of Dairy Science, Vol. 67, pages 606-613, 1984
Non-patent document 3: Bellamy, W. et al., Biochimica et Biophysica Acta, Vol. 1121, pages 130-136, 1992
Non-patent document 4: Bellamy, W. et al., Journal of Applied Bacteriology, Vol. 73, pages 472-479, 1992

Summary of the Invention

Problems to be solved by the Invention

Lactoferrin and lactoferrin hydrolysate (henceforth also collectively referred to as "lactoferrins") are known from the past to have an antimicrobial activity. And in order to further improve the antimicrobial activity of lactoferrins, methods of combining lactoferrins and various compounds have been investigated.

However, combinations that markedly enhance the antimicrobial effect of lactoferrins are scarcely known.

In particular, there has been desired a method that can enhance the antimicrobial activity of lactoferrins even with an extremely small amount of a substance to be added to lactoferrins.

Furthermore, there has been desired a highly safe antimicrobial agent that show no adverse reactions even if it is ingested on a daily basis.

Means for solving the problem

The inventors of the present invention paid attention to the fact that a protein fraction in the middle of purification obtainable in the production process of lactoferrin markedly enhanced the antimicrobial activity of lactoferrins, and conducted various researches on protein components of the fraction.

Then, they confirmed that, among the aforementioned components, ribonuclease-4 which is a milk protein not having antimicrobial activity by itself, markedly enhanced the antimicrobial activity of lactoferrin or lactoferrin hydrolysate, and accomplished the present invention.

The present invention relates to the subject-matter defined in the claims.

As the first aspect, the present invention solving the aforementioned problem provides an antimicrobial activity enhancing agent for lactoferrin or a lactoferrin hydrolysate, which contains ribonuclease-4 as an active ingredient.

The second aspect of the present invention relates to an antimicrobial composition comprising the antimicrobial activity enhancing agent of the first aspect of the present invention, and one or plural kinds of substances selected from the group consisting of lactoferrin and a lactoferrin hydrolysate, wherein mass ratio of ribonuclease-4 to lactoferrin and a lactoferrin hydrolysate is 1/80 or more.

The third aspect of the present invention relates to a drug for antimicrobial use comprising the antimicrobial composition of the second aspect of the present invention.

The fourth aspect of the present invention relates to a food or drink comprising the antimicrobial composition of the second aspect of the present invention.

The fifth aspect of the present invention relates to a feed comprising the antimicrobial composition of the second aspect of the present invention.

The sixth aspect of the present invention relates to use of ribonuclease-4 for the production of an antimicrobial activity enhancing agent for lactoferrin or a lactoferrin hydrolysate.

The seventh aspect of the present invention relates to a method for producing an antimicrobial activity enhancing agent for lactoferrin or a lactoferrin hydrolysate, which comprises:

(a) the step of contacting raw milk with a cation exchange resin to adsorb proteins in the raw milk to the cation exchange resin,
(b) the step of washing the cation exchange resin having undergone the aforementioned adsorption treatment, and flowing an elution solvent to elute a protein fraction having an isoelectric point of 8.0 to 10.0, and
(c) the step of subjecting the eluted protein fraction to gel filtration using a gel filtration carrier to collect a fraction of a molecular weight of 30 kDa or less.

The eighth aspect of the present invention relates to a method for producing ribonuclease-4, which comprises:

(a) the step of contacting a raw milk with a cation exchange resin to adsorb proteins in the raw milk to the cation exchange resin,
(b) the step of washing the cation exchange resin having undergone the aforementioned adsorption treatment, and flowing an elution solvent to elute a protein fraction having an isoelectric point of 8.0 to 10.0,
(c) the step of subjecting the eluted protein fraction to membrane filtration using an ultrafiltration membrane having a molecular weight cutoff of 30 kDa to collect a fraction passed through the membrane, and

(d) the step of subjecting the fraction passed through the membrane to gel filtration using a gel filtration carrier to collect a fraction of a molecular weight of 30 kDa or less.

Brief Description of the Drawings

[Fig. 1] A drawing (partially consisting of photograph) showing an elution curve observed in gel filtration chromatography used for the method for producing the antimicrobial activity enhancing agent of the present invention.

[Fig. 2] A drawing (partially consisting of photograph) showing an elution curve observed in gel filtration chromatography used for the method for producing the antimicrobial activity enhancing agent of the present invention.

[Fig. 3] An SDS electrophoretogram (photograph) of the antimicrobial activity enhancing agent of the present invention.

[Fig. 4] An SDS electrophoretogram (photograph) of the antimicrobial activity enhancing agent of the present invention.

[Fig. 5] The results of amino acid sequence (SEQ ID No.: 4) analysis for the antimicrobial activity enhancing agent of the present invention (ribonuclease-4) obtained by mass spectrometry. The upper and lower rows indicate the results of the analysis performed twice. The underlined parts are portions that agreed with the sequence of the bovine ribonuclease-4 in database search performed by using the results of mass spectrometry.

[Fig. 6] A densitogram (partially consisting of photograph) of the antimicrobial activity enhancing agent of the present invention.

[Fig. 7] A densitogram (partially consisting of photograph) of the antimicrobial activity enhancing agent of the present invention.

[Fig. 8] The primary structure of human Lactoferricin (amino acid sequence: SEQ ID NOS: 1 and 2).

[Fig. 9] The primary structure of bovine Lactoferricin (amino acid sequence: SEQ ID NO: 3).

Modes for Carrying out the Invention

Hereafter, preferred embodiments of the present invention will be explained in detail. However, the present invention is not limited to the following preferred embodiments, and freely changed within such a range that the present invention is feasible.

[Antimicrobial activity enhancing agent]

The antimicrobial activity enhancing agent of the present invention has an effect of enhancing the inherent antimicrobial activity of lactoferrin or a lactoferrin hydrolysate.

Although the antimicrobial activity enhancing agent of the present invention may consist only of ribonuclease-4, it may contain other components, for example, components used for formulating a drug mentioned later, so long as it contains ribonuclease-4 as an active ingredient. Further, the antimicrobial activity enhancing agent of the present invention may contain a milk protein other than ribonuclease-4 (except for lactoferrin), so long as it contains ribonuclease-4 as an active ingredient. Purity of ribonuclease-4 based on the proteins contained in the antimicrobial activity enhancing agent is preferably 0.01% or more, more preferably 0.1% or more, still more preferably 1.0% or more. The amount of lactoferrin will be explained later.

The antimicrobial activity enhancing agent of the present invention may be mixed with lactoferrin or a lactoferrin hydrolysate as liquids, or they may be mixed as powders. If they are made into powders and then mixed, operation for formulating a preparation such as tablet and troche becomes easier.

Ribonuclease-4 as the active ingredient of the antimicrobial activity enhancing agent of the present invention does not have antimicrobial activity. The antimicrobial activity enhancing agent of the present invention exhibits the effect as an antimicrobial activity enhancing agent only when it is used together with lactoferrin or a lactoferrin hydrolysate, or it is mixed with lactoferrin or the lactoferrin hydrolysate.

As the effect of lactoferrin or a lactoferrin hydrolysate containing the antimicrobial activity enhancing agent according to an embodiment of the present invention, it can reduce number of *Escherichia coli* to at least 1/19 or less of that obtainable with lactoferrin or the lactoferrin hydrolysate not containing the antimicrobial activity enhancing agent, as demonstrated in the test examples mentioned later. In other words, it can reduce amount of bacteria (for example, amount of *Escherichia coli*) represented by CFU (colony forming unit) to 1/19 or less.

That is, as for enhancement of the antimicrobial activity by the antimicrobial activity enhancing agent of the present invention, the antimicrobial activity enhancing agent of the present invention has a property that it can enhance the antimicrobial activity of lactoferrin or a lactoferrin hydrolysate of a specific amount against *Escherichia coli* 19 times or more, when the antimicrobial activity enhancing agent and lactoferrin or a lactoferrin hydrolysate are used together.

If the number of a microorganism is decreased to 1/n by a specific amount of lactoferrin or a lactoferrin hydrolysate, and the number of the microorganism is decreased to 1/m (provided that m > n) by lactoferrin or the lactoferrin hydrolysate and the antimicrobial activity enhancing agent used together, it means that the antimicrobial activity of lactoferrin or the lactoferrin hydrolysate is enhanced m/n times.

Degree of the enhancement of the antimicrobial activity is preferably evaluated under a condition providing the highest antimicrobial activity determined by adding the antimicrobial activity enhancing agent of the present invention in various amounts to a constant amount of lactoferrin or a lactoferrin hydrolysate.

The antimicrobial activity enhancing agent of the present invention can be used also as a pharmaceutical composition for enhancing antimicrobial action, an antimicrobial action enhancing agent and an additive for enhancing antimicrobial action.

In the present invention, the term "antimicrobial activity" means a concept including activities for suppressing proliferation of a microorganism (antimicrobial activity), eliminating microorganisms from an object to reduce the number thereof (decolonization activity) and killing microorganisms (sterilization activity).

Therefore, the antimicrobial activity enhancing agent of the present invention may also be referred to as decolonization adjuvant or sterilization adjuvant. Similarly, the antimicrobial activity enhancing agent of the present invention may also be referred to as decolonization composition or sterilization composition.

In the present invention, the microorganism as the object of the antimicrobial activity to be enhanced is not particularly limited, so long as it is a microorganism against which lactoferrin or a lactoferrin hydrolysate shows antimicrobial activity. Examples include gram-negative bacteria such as *Escherichia, Salmonella, Listeria, Bacillus, Cronobacter, Klebsiella* and *Pseudomonas* bacteria, gram-positive bacteria such as *Staphylococcus* and *Streptococcus* bacteria, fungi such as *Candida* yeasts, and so forth.

[Ribonuclease-4]

Ribonuclease-4 as the active ingredient of the present invention can be prepared by using colostrum, transitional milk, normal milk, late lactation milk or the like of a mammal (for example, human, cow, water buffalo, horse, goat, sheep etc.), or skim milk which is a processed product of the foregoing milks, as raw milk, and subjecting it to a cation exchange treatment, an ultrafiltration membrane treatment or a gel filtration treatment using a gel filtration carrier.

Further, ribonuclease-4 can be prepared from any raw milk containing whey (milk serum) proteins without particular limitation.

For example, bovine ribonuclease-4 is a protein consisting of 147 amino acid residues and having a molecular weight of 17 kDa and an isoelectric point of 9.18. Although ribonuclease-4 is known to have the ribonuclease activity and cytotoxicity against cancer cells, other physiological activities thereof have not been known so far.

Furthermore, ribonuclease-4 is not limited to ribonuclease-4 obtained from milk or a dairy product as raw milk, but may be a protein obtained from a body fluid other than milk or a tissue of animals, a chemically synthesized product or a recombinant protein, so long as it has a structure equivalent to ribonuclease-4. Further, ribonuclease-4 or fragments thereof are not limited to those having the amino acid sequence of SEQ ID NO: 4, but may be a conservative variant thereof. Examples of the conservative variant thereof include a protein having the amino acid sequence of SEQ ID NO: 4 including substitution, deletion, insertion, addition, inversion or the like of one or several amino acid residues, for example, 1 to 10 amino acid residues, preferably 1 to 5 amino acid residues, more preferably 1 to 3 amino acid residues, in the sequence, and a protein having a homology of 80% or more, preferably 90% or more, more preferably 95% or more, to the amino acid sequence of SEQ ID NO: 4, and having an action of enhancing the antimicrobial activity of lactoferrin or a lactoferrin hydrolysate.

[Lactoferrin]

As lactoferrin used in the present invention as the object of enhancement of antimicrobial activity, any of marketed lactoferrin, lactoferrin isolated from colostrum, transitional milk, normal milk, late lactation milk or the like of a mammal (for example, human, cow, water buffalo, horse, goat, sheep etc.), skim milk, whey etc. which are processed products of the foregoing milks and so forth by a conventional method such as ion exchange chromatography, apolactoferrin produced by deironization of lactoferrin in a conventional manner, and metal-unsaturated or saturated lactoferrin produced by partially or fully chelating apolactoferrin with a metal such as iron, copper, zinc and manganese may be used.

Further, human lactoferrin produced by a recombinant fungus, a recombinant cow (transgenic cow) or the like obtained by a recombinant DNA technique, and so forth can be similarly used for the present invention.

[Lactoferrin hydrolysate]

In the present invention, as the lactoferrin hydrolysate as the object of the enhancement of antimicrobial activity, a marketed product, or a hydrolysate obtained by hydrolyzing the lactoferrin defined in the present invention with an acid or an enzyme can be used, and it can be obtained by, for example, the method described in Japanese Patent Application No. 3-171736.

In addition, in the present invention, the lactoferrin hydrolysate as the object of the enhancement of antimicrobial activity also includes a lactoferrin peptide isolated or purified from a lactoferrin hydrolysate.

Examples of the lactoferrin peptide include, for example, Lactoferricin (registered trademark), and examples of Lactoferricin include, for example, human Lactoferricin (Lactoferricin H) and bovine Lactoferricin (Lactoferricin B) .

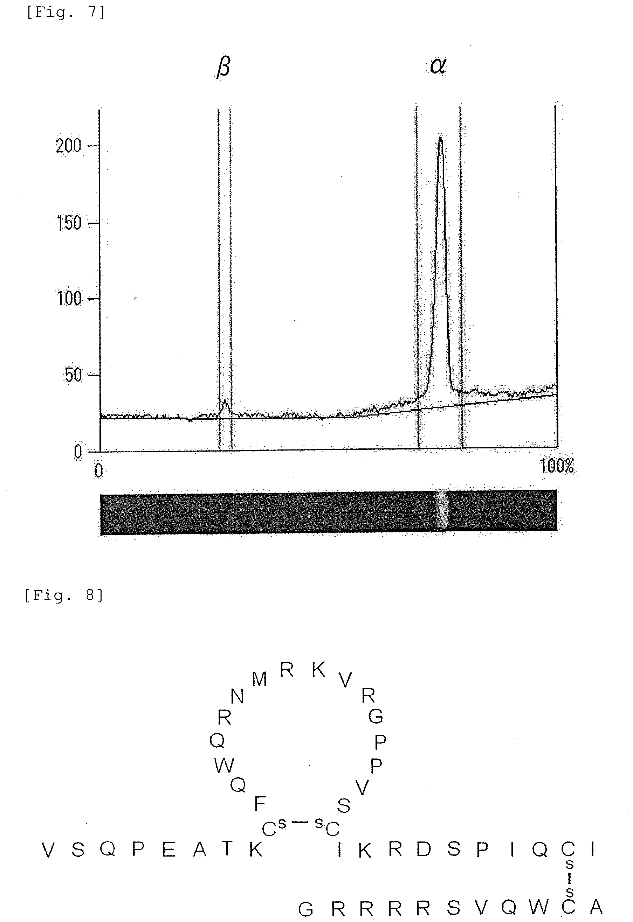

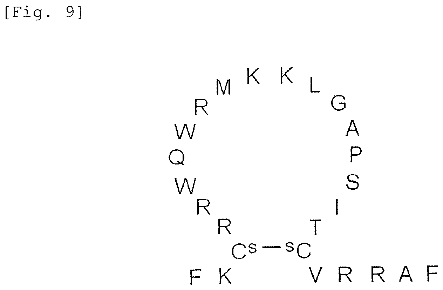

Fig. 8 shows the primary structure of human Lactoferricin (Lactoferricin H) (amino acid sequence: SEQ ID NOS: 1 and 2), and Fig. 9 shows the primary structure of bovine Lactoferricin (Lactoferricin B) (amino acid sequence: SEQ ID NO: 3).

When lactoferrin is hydrolyzed with an acid, lactoferrin is made into an aqueous solution, an inorganic acid or an organic acid is added to the solution, and the mixture is heated at a predetermined temperature for a predetermined time to hydrolyze lactoferrin. When the hydrolysis is performed with an enzyme, lactoferrin is made into an aqueous solution, the solution is adjusted to the optimum pH of an enzyme to be used, an enzyme such as pepsin and trypsin is added to the solution, and the mixture is maintained at a predetermined temperature for a predetermined time to perform the hydrolysis. After the hydrolysis, the enzyme is inactivated in a conventional manner. The hydrolysate obtained by the hydrolysis with an acid or enzyme is a mixture of hydrolysate of various molecular weights containing a peptide having antimicrobial activity. Degree of the decomposition by the aforementioned hydrolysis is desirably in the range of 6 to 20%. This degree of decomposition is calculated in accordance with the following equation from total nitrogen amount in a sample measured by the Kjeldahl method, and formol nitrogen amount in the sample measured by the formol titration method.

```
Degree of decomposition = (Formol nitrogen amount/Total
nitrogen amount) x 100
```

The reaction mixture obtained by the aforementioned hydrolysis with an acid or enzyme (solution of lactoferrin hydrolysates) is cooled in a conventional manner, neutralized, desalted and decolorized in a conventional manner as required, and further fractionated as required, and the obtained solution as it is, or as a concentrate obtained by concentrating the solution, or powder obtained by concentrating the solution and lyophilizing the residue is used. Further, as described above, an antimicrobial peptide such as Lactoferricin may be isolated from the hydrolysate.

[Active ingredient of the present invention]

The active ingredient of the antimicrobial activity enhancing agent of the present invention consists of ribonuclease-4, and this component has an action and effect of enhancing the antimicrobial activity of lactoferrin or a lactoferrin hydrolysate.

As the inventors of the present invention find the effect of ribonuclease-4, the inventors elucidated at least that 1) the protein as the component that enhances the antimicrobial activity does not show antimicrobial activity by itself, 2) the protein has a molecular weight of 10 to 30 kDa, and 3) the protein has an isoelectric point of 8.0 to 10.0. And they finally found that the active ingredient is ribonuclease-4.

Isoelectric point of ribonuclease-4 expected from the amino acid sequence is 9.18.

Ribonuclease-4 may be a fragment thereof so long as it is a fragment having the action of enhancing the antimicrobial activity of lactoferrin or a lactoferrin hydrolysate.

Although it is preferred that the aforementioned protein that can serve as the active ingredient of the present invention (ribonuclease-4) does not contain lactoferrin or a lactoferrin hydrolysate at all, it has been confirmed that, if ratio of lactoferrin or a lactoferrin hydrolysate in ribonuclease-4 as the active ingredient is 4.0% or less in terms of mass ratio, the protein as a whole does not show antimicrobial activity.

In addition, the inventors of the present invention also found that angiogenins (Angiogenin-1 and Angiogenin-2) as milk proteins have an action of enhancing the antimicrobial activity of lactoferrin or a lactoferrin hydrolysate. The active ingredient of the antimicrobial activity enhancing agent of the present invention may contain angiogenin in addition to ribonuclease-4.

[Amount of antimicrobial activity enhancing agent]

When the antimicrobial activity enhancing agent of the present invention is used for lactoferrin or a lactoferrin hydrolysate, mass ratio of ribonuclease-4 in the antimicrobial activity enhancing agent and lactoferrin or a lactoferrin hydrolysate is preferably 1:80 to 1:1, more preferably 1:80 to 1:10, still more preferably 1:60 to 1:10, further preferably 1:40 to 1:10.

Further, when the antimicrobial activity enhancing agent of the present invention is used together with, or mixed with a lactoferrin hydrolysate, in particular with Lactoferricin (registered trademark), which is a peptide derived from lactoferrin, they are preferably used at a ratio of ribonuclease-4 in the antimicrobial activity enhancing agent and Lactoferricin of 1:1 to 4:1, more preferably at a ratio of ribonuclease-4 in the antimicrobial activity enhancing agent and Lactoferricin of 1.5:1 to 2.5:1.

[Antimicrobial agent composition]

The antimicrobial composition of the present invention contains the antimicrobial activity enhancing agent of the present invention, and one or plural kinds of substances selected from the group consisting of lactoferrin and a lactoferrin hydrolysate, and is characterized in that mass ratio of ribonuclease-4 in the antimicrobial activity enhancing agent to lactoferrin and the lactoferrin hydrolysate is 1/80 or more. Although the mass ratio of ribonuclease-4 to lactoferrin and the lactoferrin hydrolysate is not particularly limited so long as it is 1/80 or more, it is preferably 1/60 or more, more preferably 1/40 or more. Although the maximum mass ratio of ribonuclease-4 in the antimicrobial activity enhancing agent to lactoferrin and the lactoferrin hydrolysate is not particularly limited, high antimicrobial activity can be expected even with a ratio of, for example, 1/1 or less, or 1/10 or less.

Mass ratio of ribonuclease-4 and lactoferrin contained in ordinary whey is about 1:200.

The antimicrobial agent composition of the present invention can be blended in drugs, foods or drinks, feeds, cosmetics, quasi drugs, and so forth.

[Drug]

The drug of the present invention is characterized by containing the antimicrobial composition of the present invention or the antimicrobial activity enhancing agent of the present invention, and lactoferrin or a lactoferrin hydrolysate.

Form of the drug of the present invention is not particularly limited, and it can be formulated in various forms by known methods, and administered. In a preferred embodiment, it can be orally administered. When the drug is formulated, the antimicrobial activity enhancing agent of the present invention, and lactoferrin or a lactoferrin hydrolysate are mixed and used.

For example, the drug can be formulated with the antimicrobial activity enhancing agent of the present invention and lactoferrin or a lactoferrin hydrolysate by using an arbitrary additive such as a pharmacologically acceptable excipient.

When the drug is formulated, content of lactoferrin or a lactoferrin hydrolysate in the drug is not particularly limited. Although it is basically considered that the antimicrobial activity enhancing agent of the present invention and lactoferrin or a lactoferrin hydrolysate scarcely show side reaction, content of the composition comprising the antimicrobial activity enhancing agent and lactoferrin or a lactoferrin hydrolysate in the drug is usually 0.1 to 60 mass %, preferably 10 to 50 mass %. Further, content of the composition comprising the antimicrobial activity enhancing agent and Lactoferricin (registered trademark) in the drug is usually 0.01 to 6 mass %, preferably 1 to 5 mass %.

The ratio of amounts of the antimicrobial activity enhancing agent and lactoferrin and a lactoferrin hydrolysate is the same as that described for the antimicrobial composition.

When the drug is formulated, additives such as excipient, binder, disintegrant, lubricant, stabilizer, corrigent, diluent and solvent for injections can be used. Specific examples of the formulation include tablets (including sugar-coated tablets, enteric coated tablets and buccal tablets), powders, capsules (including enteric capsules and soft capsules), granules (including coated granules), pills, troches, enclosed liposome agents, solutions, pharmaceutically acceptable sustained release preparations of these, and so forth.

Examples of the carrier and excipient used for these formulations include lactose, glucose, sucrose, mannitol, potato starch, corn starch, calcium carbonate, calcium phosphate, calcium sulfate, crystalline cellulose, glycyrrhizae radix pulverata, gentianae radix pulverata, and so forth, and examples of the binder include starch, gelatin, syrup, polyvinyl alcohol, polyvinyl ether, polyvinylpyrrolidone, hydroxypropylcellulose, ethylcellulose, methylcellulose, carboxymethylcellulose, and so forth.

Examples of the disintegrant include starch, agar, gelatin powder, carboxymethylcellulose sodium, carboxymethylcellulose calcium, crystalline cellulose, calcium carbonate, sodium hydrogencarbonate, sodium arginate, and so forth.

Examples of the lubricant include magnesium stearate, hydrogenated vegetable oil, Macrogol, and so forth, and examples of the colorant include Red No. 2, Yellow No. 4, Blue No. 1, and so forth, which are allowed to be added to drugs.

Tablets and granules can be coated with sucrose, hydroxypropylcellulose, purified shellac, gelatin, sorbitol, glycerol, ethylcellulose, hydroxypropylmethylcellulose, polyvinylpyrrolidone, cellulose acetate phthalate, hydroxypropylmethylcellulose phthalate, methyl methacrylate, methacrylic acid polymer, and so forth, as required.

Further, the pharmaceutical composition used together may be contained in the drug of the present invention as an active ingredient, or may not be contained in the drug of the present invention, but may be prepared as a separate drug and combined with the drug to provide a commercial product.

Examples of the pharmaceutical composition for antimicrobial use include drugs and quasi drugs such as dentifrice, and mouthwash.

[Food and drink]

The food or drink of the present invention is characterized by containing the antimicrobial composition of the present invention or the antimicrobial activity enhancing agent of the present invention, and lactoferrin or a lactoferrin hydrolysate.

Content of the composition comprising the antimicrobial activity enhancing agent and lactoferrin or a lactoferrin hydrolysate in the food or drink of the present invention is usually 0.1 to 60 mass %, preferably 10 to 50 mass %. Content of the composition comprising the antimicrobial activity enhancing agent and Lactoferricin (registered trademark) in the food or drink is usually 0.01 to 6 mass %, preferably 1 to 5 mass %.

Ratio of amounts of the antimicrobial activity enhancing agent and lactoferrin and a lactoferrin hydrolysate is the same as that explained for the antimicrobial composition.

Form of the food or drink of the present invention is not particularly limited, and examples include soft drinks, milk beverages etc. or concentrated undiluted solutions or powders for preparation of these drinks; dairy products such as processed milk and fermented milk; processed milk powder for baby; foods for enteral feeding; functional foods; and so forth, as well as drinks such as carbonated drinks, nutritious drinks and fruit drinks (including concentrated undiluted solutions or powders for preparation of these drinks); frozen desserts such as ice cream, ice sherbet and chipped ice; noodles such as buckwheat noodles, udon, bean thread noodles, wrapping sheets for Chinese meat dumpling, wrapping sheets for steamed meat dumpling, Chinese noodles and instant noodles; confectioneries such as candy, chewing gum, candy, chocolate, tablet confectioneries, snacks, biscuits, jelly, jam, cream and baked confectioneries; aquatic and livestock processed foods such as boiled fish paste, ham and sausage; fats, oils, and fat or oil processed foods such as salad oil, tempura oil, margarine, mayonnaise, shortening, whipped cream and dressing; seasonings such as sauce and dipping sauce; soup, stew, salad, daily dish, pickles, bread, and so forth, in which powder of lactoferrin or a lactoferrin hydrolysate or an aqueous solution thereof (syrup etc.) is formulated together with the antimicrobial activity enhancing agent of the present invention.

Such foods and drinks can be produced by, for example, blending lactoferrin (including lactoferrin in the form of lactoferrin powder, lactoferrin aqueous solution (syrup etc.), and so forth) with the antimicrobial activity enhancing agent of the present invention, as well as saccharides such as dextrin and starch; proteins such as gelatin, soybean protein and corn protein; amino acids such as alanine, glutamine and isoleucine; polysaccharides such as cellulose and gum arabic; oil or fat such as soybean oil and medium chain fatty acid triglyceride, and so forth.

Further, preferred examples of the form of the food or drink include supplement in the form of a liquid or tablet, and so forth.

[Feed]

The feed of the present invention is characterized by containing the antimicrobial composition of the present invention or the antimicrobial activity enhancing agent of the present invention and lactoferrin or a lactoferrin hydrolysate.

Content of the composition comprising the antimicrobial activity enhancing agent and lactoferrin or a lactoferrin hydrolysate in the feed of the present invention is usually 0.1 to 60 mass %, preferably 10 to 50 mass %. Content of the composition comprising the antimicrobial activity enhancing agent and Lactoferricin (registered trademark) in the feed is usually 0.01 to 6 mass %, preferably 1 to 5 mass %.

Ratio of amounts of the antimicrobial activity enhancing agent and lactoferrin or a lactoferrin hydrolysate is the same as that explained for the antimicrobial composition.

Form of the feed of the present invention is not particularly limited. For example, powder of lactoferrin or a lactoferrin hydrolysate or an aqueous solution thereof (syrup etc.) can be blended together with the antimicrobial activity enhancing agent of the present invention, and the feed can be produced by, for example, blending the antimicrobial activity enhancing agent of the present invention and lactoferrin with cereals such as corn, wheat, barley, rye and milo; vegetable oil meals such as soybean oil meal, rapeseed oil meal, coconut oil meal and linseed oil meal; brans such as bran, wheat bran, rice bran and defatted rice bran; production meals such as cone gluten meal and corn jam meal; animal-derived feeds such as fish meal, skim milk powder, whey, yellow grease and tallow; yeasts such as torula yeast and brewer's yeast; mineral material feeds such as calcium triphosphate and calcium carbonate; oils and fats; simple substance amino acids; saccharides, and so forth. Examples of the form of the feed include pet food, livestock feed, fish breeding feed, and so forth.

[Method for producing ribonuclease-4]

Ribonuclease-4 as the active ingredient of the antimicrobial activity enhancing agent of the present invention can be produced by, for example, the method comprising the following steps using a raw material derived from milk as a starting material.

That is, it is a method comprising (a) the step of contacting raw milk with a cation exchange resin to adsorb proteins in the raw milk to the cation exchange resin, (b) the step of washing the cation exchange resin having undergone the aforementioned adsorption treatment, and flowing an elution solvent to elute a protein fraction having an isoelectric point of 8.0 to 10.0, (c) the step of subjecting the eluted protein fraction to membrane filtration using an ultrafiltration membrane having a molecular weight cutoff of 30 kDa to collect a fraction passed through the membrane, and (d) the step of subjecting the fraction passed through the membrane to gel filtration using a gel filtration carrier to collect a fraction of a molecular weight of 30 kDa or less.

The production method of the present invention may further comprise, following the aforementioned step of (d), (e) the step of flowing the fraction of a molecular weight of 30 kDa or less through an ultrafiltration membrane having a molecular weight cutoff of 10 kDa to collect a concentrate of a fraction of 10 kDa or more.

When the antimicrobial activity enhancing agent of the present invention is used for enhancing the antimicrobial activity of lactoferrin, a lactoferrin hydrolysate, or a lactoferrin-derived peptide, a fraction of ribonuclease-4 produced by the aforementioned method in which the step of (c) is not performed, and the step of (d) is performed immediately after the step of (b) can be used as the antimicrobial activity enhancing agent of the present invention.

In such a case, it is sufficient that ribonuclease-4 as the active ingredient has a protein purity of 96 mass % or more, and if content of lactoferrin as the other component is 4.0 mass % or less, the produced antimicrobial activity enhancing agent per se does not substantially show antimicrobial activity.

As a reason for this, it has been confirmed that a fraction collected after the aforementioned step of (b) contains lactoferrin derived from milk together with ribonuclease-4, but a fraction of ribonuclease-4 produced by the method in which the step of (c) is not performed, and the step of (d) is performed immediately after the step of (b) contains lactoferrin in an amount of 4 mass % or less based on the amount of total proteins, as shown in Test Example 1 mentioned later. And, it has also been confirmed that even if such a small amount of lactoferrin is contained, the produced fraction of ribonuclease-4 does not show antimicrobial activity against bacteria such as *Escherichia coli* by the fraction itself.

That is, when the antimicrobial activity enhancing agent of the present invention is used for enhancing the antimicrobial activity of lactoferrin, a lactoferrin hydrolysate or a lactoferrin-derived peptide, even if a fraction of ribonuclease-4 produced by the method in which the step of (c) is not performed, and the step of (d) is performed immediately after the step of (b) is used as the antimicrobial activity enhancing agent of the present invention, enhancement of the antimicrobial activity of lactoferrin, a lactoferrin hydrolysate or a lactoferrin-derived peptide is not affected.

On the other hand, it has been found that a fraction of ribonuclease-4 produced by the method in which the step of (c) is performed immediately after the step of (b), and then the step of (d) is performed has a protein purity of about 100%.

As elucidated during the investigation for the method for producing the antimicrobial activity enhancing agent of the present invention as described above, ribonuclease-4 can be produced with high purity from a raw material derived from milk by the method comprising the aforementioned steps (a) to (d). Further, by performing the aforementioned method, ribonuclease-4 having a markedly improved purity can be produced, as compared with that obtainable by a conventional method for producing ribonuclease-4.

Therefore, the production method of the present invention also has an effect that it can provide ribonuclease-4 of high purity for various uses, also in the case that ribonuclease-4 is used for uses other than the antimicrobial activity enhancing agent of the present invention.

The method for producing ribonuclease-4 as the active ingredient of the antimicrobial activity enhancing agent of the present invention partially overlaps with a method for producing lactoferrin or lactoperoxidase from a conventional raw material derived from milk, but it is different in that it comprises the step of (c) or (d). That is, since only low molecular weight proteins of 30 kDa or less are collected, lactoferrin or lactoperoxidase, each of which has a molecular weight of about 80,000 daltons, are scarcely contained in the antimicrobial activity enhancing agent of the present invention.

The method for producing the antimicrobial activity enhancing agent of the present invention will be further explained in detail below.

First, raw milk is contacted with a cation exchanger to perform an adsorption treatment.

The raw milk is not particularly limited so long as a material containing a protein having the antimicrobial activity enhancing action is selected, and examples include raw milk of a mammal (for example, human, cow, water buffalo, horse, goat, sheep etc.), processed milk such as skim milk, fractionation products such as whey, milk produced by a recombinant cow (transgenic cow) or the like, and so forth. The milk may be any of colostrum, transitional milk, normal milk, late lactation milk, and so forth.

As the cation exchanger, one having a weakly acidic group or a strongly acidic group as the ion exchange group is used. As the weakly acidic ion exchange group or the strongly acidic ion exchange group, an ion exchange group having an objective function as an ion exchange group can be arbitrarily selected. Examples of the weakly acidic ion exchange group include carboxyl group, carboxymethyl group, and so forth. Examples of the strongly acidic ion exchange group include sulfopropyl group, and so forth.

The cation exchanger is not particularly limited, and can be arbitrarily selected as required. Preferred examples of the cation exchanger include those comprising porous particles consisting of a cross-linked polysaccharide (agarose, dextran, cellulose etc.), hydrophilic silica gel, synthetic polymer, or the like, introduced with weakly acidic or strongly acidic ion exchange groups. Specific examples of the weakly acidic ion exchanger include Sepabeads FP-CM13 (Mitsubishi Chemical, exchange group: carboxymethyl group), CM-Sephadex C-50 (GE Healthcare, exchange group: carboxymethyl group), and CM-Sepharose FF (GE Healthcare, exchange group: carboxymethyl group). Specific examples of the strongly acidic ion exchanger include SP-Sepharose FF (GE Healthcare) and SP-Sepharose Big Beads (GE Healthcare).

Form, size, surface condition, material etc. of the resin of the cation exchanger are not limited, and may be arbitrarily selected as required. Examples of the form of the cation exchanger for use include a pre-packed column already filled with an ion exchanger resin, a medium such as column filled with resin beads of a cation exchanger, and so forth. When these forms are used, it is preferred that one kind of cation exchanger is used in combination with one kind of medium in view of simplicity. However, two or more media each filled with resin beads may be connected in series or in parallel to perform chromatography, if needed. Although the form of the medium may be arbitrarily selected as required, a form allowing easy washing thereof and providing a larger contact area for resin beads or the like to be contained is preferred, and internal surface thereof is preferably a flat surface having no unevenness.

Specifically, a shape having a circularly curved side such as cylindrical shape (cylindrical column, rod) and conical shape is preferably used as the shape of the medium. The material of the medium may be arbitrarily selected, and stainless steel, glass, polypropylene, polyethylene, polyethylene terephthalate, polycarbonate, acrylic resin and for forth can be preferably used. Size of the medium may be appropriately selected depending on the scale of the process, and it can be arbitrarily selected from the range of, for example, several cubic centimeters to several cubic meters.

Examples of commercially available pre-packed column that can be preferably used for the present invention include Hiprep CMFF16/10 (GE Healthcare, exchange group: carboxymethyl group), Hiprep SPFF16/10 (GE Healthcare, exchange group: sulfopropyl group), TOYOPEARL CM-650 Series (TOSOH, exchange group: carboxymethyl group), TOYOPEARL SP-650 Series (TOSOH, exchange group: sulfopropyl group), and so forth.

The method of the adsorption treatment (contact) for the raw milk and the cation exchanger may be arbitrarily selected. The adsorption treatment can be performed by such a method as the batch type stirring method and continuous column method. Any method may be employed so long as the raw milk and the cation exchanger can fully be contacted. The adsorption treatment may consist of one adsorption treatment, or a combination of two or more adsorption treatments.

In the case of batch type process, it is appropriate to use a large amount of the cation exchanger when high yield is desired from a certain fixed amount of the raw milk, or to use a large amount of the raw milk when it is desired to obtain high yield by using a certain fixed amount of the cation exchanger. The mixing volume ratio of the raw milk and the cation exchanger in the case of batch type process can be arbitrarily adjusted depending on adsorption ability of the cation exchanger and type of specific method used for the adsorption treatment.

Cation exchangers are roughly classified into two types, those of hard type and soft type, according to the characteristics thereof. In the present invention, those of both types can be used.

Cation exchangers of the hard type hardly show change of volume of the cation exchangers themselves depending on ionic strength or pH, and also hardly show change of volume of the cation exchangers themselves even if pressure applied to the cation exchangers is changed with change of flow rate or the like. Therefore, cation exchangers of the hard type are more suitable for the continuous column method in which a cation exchanger is retained in a column, and a solution is flown through it at a high flow rate.

On the other hand, cation exchangers of the soft type show significant change of volume of the cation exchangers themselves depending on ionic strength or pH, and also easily show change of volume of the cation exchangers themselves when pressure applied to the cation exchangers is changed with change of flow rate or the like. Therefore, it is difficult to retain a cation exchanger of the soft type in a column, and flow a solution through it at a high flow rate. In particular, when skim milk, whey or the like is flown through the cation exchanger, a larger pressure loss occurs in a cation exchanger layer compared with the case of flowing other salt solutions through it. Therefore, cation exchangers of the soft type are generally suitable for the batch type process.

Among those mentioned above, Sepabeads FP-CM13 (Mitsubishi Chemical), CM-Sepharose FF (GE Healthcare), SP-Sepharose FF (GE Healthcare) and SP-Sepharose Big Beads (GE Healthcare) are cation exchangers of the hard type, and CM-Sephadex C-50 (Amersham) is a cation exchanger of the soft type.

If the adsorption treatment of the raw milk and the cation exchanger is performed at a temperature lower than 0°C, the raw milk may be frozen, and viscosity thereof may be increased, and if it is performed at a temperature exceeding 60°C, proteins in the milk, for example, basic proteins in the milk, may be denatured. Therefore, the treatment is preferably performed at a temperature in the range of 0 to 60°C. In addition, even within such a range, when the treatment is performed at a temperature of 25 to 60°C, if the adsorption treatment takes a long time, proteins in the milk, for example, basic proteins in the milk, may be gradually denatured. Therefore, the treatment is especially preferably performed at a temperature of 0 to 25°C. Further, when unsterilized raw milk is used, the treatment is preferably performed at a temperature of 0 to 10°C in order to prevent proliferation of bacteria.

Time of the adsorption treatment (contact time) of the raw milk and the cation exchanger may be appropriately selected, in consideration of temperature for the adsorption treatment, method of the adsorption treatment (batch type process or continuous column method) to be employed, etc. For example, in the case of the batch type process, time of the adsorption treatment of the raw milk and the cation exchanger is preferably 1 minute or more to 24 hours or less, more preferably 10 minutes or more to 6 hours or less. Further, in the case of the continuous column method, linear flow rate is preferably 10 cm/h or more to 1000 cm/h or less.

Then, the cation exchanger having undergone the adsorption treatment is subjected to a washing treatment. As a washing solution for this treatment, a salt solution having a low ionic strength lower than 0.07 or a neutral or weakly acidic buffer may be used. However, in view of the production cost, washing is preferably performed with water.

The raw milk used may be removed from the cation exchanger having undergone the adsorption treatment by any washing method. For example, only the cation exchanger is transferred from a container containing the raw milk and the cation exchanger, and washed in another place, or the raw milk may be removed from such a container as mentioned above, and then the cation exchanger may be washed in the container.

Then, an elution solvent is contacted with the cation exchanger having undergone the washing treatment to elute a fraction containing ribonuclease-4.

Although ionic strength of the elution solvent used in this step is not particularly limited, a solvent having an ionic strength 0.07 or more to 1.7 or less is preferably used. An elution fraction having an isoelectric point of 8.0 to 10.0 can be thereby obtained.

As the elution solvent, a neutral or weakly acidic buffer of which ionic strength is adjusted to be within the aforementioned range can also be used. However, it is more preferable to use an aqueous solution dissolving only salts (salt solution), if the production cost is taken into consideration. As the salt used for the present invention, one or plural kinds of salts may be arbitrarily selected as required.

Preferred salt solution is a solution of a salt containing one kind of salt or a mixture of two or more kinds of salts selected from the group consisting of sodium chloride, potassium chloride, calcium chloride, magnesium chloride, and so forth, and specifically, 1 to 5% solution of sodium chloride, preferably 1.5 to 3.5% solution of sodium chloride, and so forth can be exemplified.

Then, the obtained eluate is subjected to a membrane treatment performed by a membrane separation method using an ultrafiltration membrane having a molecular weight cutoff of 30 kDa, and the fraction passed through the membrane is collected.

Operation methods for ultrafiltration membranes are classified into two types, i.e., the usual ultrafiltration in which water and components having a molecular weight below the molecular weight cutoff are passed through the membrane to attain the removal, and the running water filtration method (diafiltration) in which the operation is continuously performed with adding water in a volume equal to the solution passed through the membrane to the solution retained on the membrane. For the present invention, both of the methods can be used. In particular, the latter running water filtration method is more preferred, because a desalting treatment can be performed simultaneously with concentration, and low molecular weight components can be fully removed from the retained solution.

When the former usual ultrafiltration method is used, it is preferable to perform a desalting treatment by such a method as dialysis or gel filtration, after the ultrafiltration.

As the ultrafiltration membrane, any of commercially available ultrafiltration membranes can be used. Specific examples include IRIS3038 and IRIS3072 membranes (both from Rhone-Poulenc S.A.), GR-61pp membrane (DDS), and so forth, and all of these have a property of a fractionation molecular weight of 30 kDa (molecular weight cutoff of 30 kDa).

As the material of the ultrafiltration membrane, any of organic materials and inorganic materials may be used, and the material can be selected in consideration of cost, availability etc.

The ultrafiltration treatment can be performed at any temperature of elution solution so long as it is lower than heat resistant temperature of the membrane used (for example, 80°C or less in the case of GR-61pp membrane). However, in order to prevent denaturation of proteins, it is preferably performed at 0 to 60°C, for example, 25 to 60°C, or 0 to 25°C.

The ultrafiltration may be performed at any pressure lower than pressure resistance limit of the membrane used (for example, 0.6 Mpa or less in the case of the GR-61pp membrane). However, use of the membrane at a pressure near the pressure resistance limit may shorten the life of the membrane, and therefore the treatment is preferably performed at a pressure 2/3 or less of the pressure resistance limit (for example, 0.4 MPa in the case of the GR-61pp membrane).

Any of forms of ultrafiltration membrane modules, for example, those of tube type, hollow fiber type, flat membrane type, spiral type, etc., may be used. When an internal pressure type hollow fiber module is used, generation of precipitates in hollow fibers may cause obstruction of flow paths, and therefore the treatment is preferably performed in consideration of the pressure etc.

Then, the fraction passed through the membrane obtained from the membrane treatment by separation with an ultrafiltration membrane is subjected to separation based on the gel filtration method.

As the gel filtration carrier used for the gel filtration treatment in the present invention, any of commercially available gel filtration resins having a molecular weight cutoff in the range of 100 kDa or less, preferably 50 kDa or less, can be used. Specific examples include TSKgel G3000SW, TSKgel G2000SW (both from TOSOH), and so forth. When the gel filtration is performed by using such a gel filtration carrier as mentioned above, it is preferable to perform the gel filtration by using a system for high speed liquid chromatography (HPLC) or the like.

As the mobile phase used for the gel filtration treatment, it is preferable to use a buffer such as citrate buffer or phosphate buffer, or a buffer comprising the above-mentioned buffer of which ionic strength is increased by dissolving about 0.5 M of a salt. It is preferred that pH of the buffer is pH suitable for characteristics of the resin used as the gel filtration carrier.

Examples

Hereafter, the present invention will be explained in detail with reference to examples and test examples.

The present invention relates to the subject-matter defined in the claims.

First, measurement methods and test methods used in the examples and the test examples will be explained.

[SDS-PAGE (SDS gel electrophoresis)]

In order to determine component composition of the antimicrobial activity enhancing agent of the present invention, SDS-PAGE was performed by using NuPAGE 4-12% Bis-Tris Gel (Invitrogen). Staining was performed by using SimplyBlue SafeStain (Invitrogen).

[Mass spectrometry]

The stained gel obtained from the SDS-PAGE was imaged with Densitograph (ATTO), and bands were confirmed by using analysis software. The obtained spots were excised from the gel, the objective protein was decolorized, reductively alkylated, digested in the gel by trypsinization, and subjected to mass spectrometry using a MALDI-TOF/MS apparatus (trade name: AutoflexII, BRUKER).

On the basis of the measurement results, the protein was identified by searching the database of NCBI (National Center for Biotechnology Information, http://www.ncbi.nlm.nih.gov/) using the MASCOT software produced by Matrix Science.

[Antimicrobial activity enhancement test]

In the antimicrobial activity enhancement test, antimicrobial activity of a sample obtained by mixing the antimicrobial activity enhancing agent of the present invention with lactoferrin, a lactoferrin hydrolysate, or the like was measured. As a control, antimicrobial activity of a sample containing only lactoferrin, a lactoferrin hydrolysate, or the like was also measured.

Specific procedure of the test was, for example, as follows.

(1) Preparation of sample

At least the following samples were prepared. For the test of lactoferrin, two kinds of Samples 2 having different concentrations of the antimicrobial activity enhancing agent were prepared.

Sample 1: Lactoferrin, lactoferrin hydrolysate, or the like
Sample 2: Mixture of lactoferrin, lactoferrin hydrolysate, or the like and antimicrobial activity enhancing agent
Sample 3: Antimicrobial activity enhancing agent

By using 1% Bacto peptone medium, *Escherichia coli* K-12 strain was inoculated into the medium contained in a polystyrene tube with a 5 ml capacity, and precultured in an incubator at 37°C for 6 hours to obtain a cell suspension for the test.

(2) Test method

The precultured *Escherichia coli* was added to a 96-well plate at a cell density of $10^6$ cells/mL together with each of the aforementioned samples, and cultured at 37°C for a predetermined time. After the culture for the predetermined time, the culture medium containing *Escherichia coli* was serially diluted with 0.85% physiological saline, and plated on an agar medium, and culture was performed at 37°C for a predetermined time. Then, number of colonies (colony forming units : CFU) was measured.

In addition, *Escherichia coli* was cultured only with the medium as a control, besides the culture using the samples examined above.

(3) Results

Antimicrobial activity of each sample was evaluated on the basis of the value of CFU. Degree of enhancement of the antimicrobial activity was calculated by comparison of the numbers of *Escherichia coli* (CFU) that remained after the test. For example, if CFU of Sample 1 is 100, and CFU of Sample 2 is 10 after the test, the antimicrobial activity of Sample 2 can be determined to be 10 times that of Sample 1.

<Example 1>

The antimicrobial activity enhancing agent of the present invention was produced.

As the cation exchange resin, CM-Sephadex C-50 (GE Healthcare) was used. 20 L of unsterilized skim milk was flown through a column filled with 170 mL of the cation exchange resin to adsorb milk proteins on the cation exchange resin.

Then, the cation exchange resin in the column was sufficiently washed with deionized water, and 0.27 M sodium chloride solution (Kokusan Chemical) was flown through the column to elute the proteins adsorbed on the resin. Subsequently, 1.7 M sodium chloride solution was flown as an elution solvent to elute a fraction of the proteins adsorbed on the resin, and an eluate in which the protein fraction was eluted was collected.

This eluate was subjected to centrifugation (4,000 x g, 15 minutes) using a centrifugal ultrafiltration membrane (trade name: Amicon Ultra, Millipore) having a molecular weight cutoff of 30 kDa, and a fraction passed through the membrane was collected.

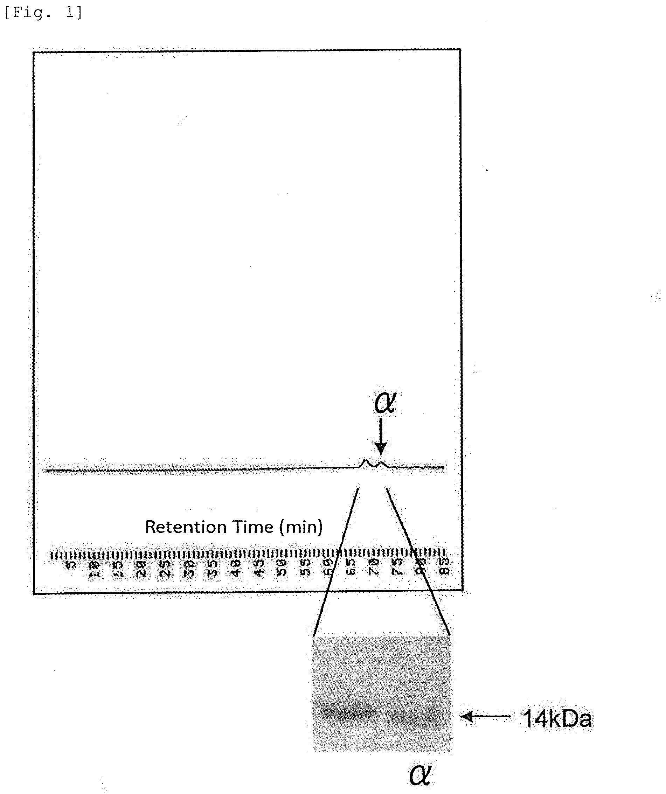

Further, the fraction passed through the membrane was dissolved in a citrate buffer (pH 5.2), and subjected to separation by HPLC using a gel filtration carrier TSKgel G2000SW (TOSOH) to separate a peak α corresponding to a molecular weight of about 14 kDa shown in Fig. 1. Fig. 1 shows the elution curve obtained in the gel filtration HPLC.

Then, the peak was desalted and concentrated with a centrifugal ultrafiltration membrane having a molecular weight cutoff of 10 kDa (trade name: Amicon Ultra, Millipore), and the fraction of the concentrated solution was collected to produce the antimicrobial activity enhancing agent of the present invention.

The molecular weight distribution of the produced antimicrobial activity enhancing agent was 10 to 30 kDa, and isoelectric point of the same was 8.0 to 10.0. On the basis of the results of amino acid sequence analysis, it was revealed that the main component of the antimicrobial activity enhancing agent was ribonuclease-4.

<Example 2>

The antimicrobial activity enhancing agent of the present invention was produced.

As the cation exchange resin, CM-Sephadex C-50 (GE Healthcare) was used. 20 L of unsterilized skim milk was flown through a column filled with 170 mL of the cation exchange resin to adsorb milk proteins on the cation exchange resin.

Then, the cation exchange resin in the column was sufficiently washed with deionized water, and 0.27 M sodium chloride solution (Kokusan Chemical) was flown through the column to elute the proteins adsorbed on the resin. Subsequently, 1.7 M sodium chloride solution was flown as an elution solvent to elute a fraction of the proteins adsorbed on the resin, and an eluate in which the protein fraction was eluted was collected.

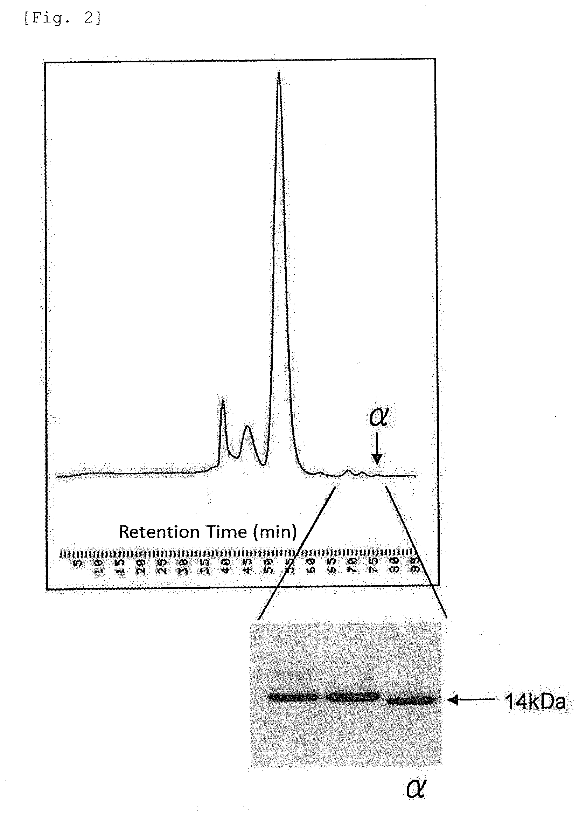

This eluate was diluted in a citrate buffer (pH 5.2) containing 0.5 M sodium chloride, and subjected to separation by HPLC using a gel filtration carrier TSKgel G2000SW (TOSOH) to separate a peak α corresponding to a molecular weight of about 14 kDa shown in Fig. 2. Fig. 2 shows the elution curve obtained in the gel filtration HPLC.

Further, the peak was desalted and concentrated with a centrifugal ultrafiltration membrane having a molecular weight cutoff of 10 kDa (trade name: Amicon Ultra, Millipore), and the fraction of the concentrated solution was collected to produce the antimicrobial activity enhancing agent of the present invention.

The molecular weight distribution of the produced antimicrobial activity enhancing agent was 10 to 30 kDa, and isoelectric point of the same was 8.0 to 10.0. On the basis of the results amino acid sequence analysis, it was revealed that the main component of the antimicrobial activity enhancing agent was ribonuclease-4.

<Example 3>

To 95 g of the bovine lactoferrin (Morinaga Milk Industry), 5 g of an antimicrobial activity enhancing agent produced in the same manner as that of Example 1 was added, and they were uniformly mixed to prepare a drug for antimicrobial use in which the antimicrobial activity of lactoferrin was enhanced.

<Example 4>

To 95 g of the bovine lactoferrin (Morinaga Milk Industry), 5 g of an antimicrobial activity enhancing agent produced in the same manner as that of Example 2 was added, and they were uniformly mixed, and the mixture was added in an amount of 10 mass % to fermented milk to prepare a fermented milk food.

Hereafter, physical properties and antimicrobial activity enhancing action of the antimicrobial activity enhancing agent of the present invention will be explained in detail with reference to test examples.

[Test Example 1]

In Test Example 1, physical properties of the components of the antimicrobial activity enhancing agents produced in Examples 1 and 2 were examined.

1) Analysis by SDS-PAGE

In order to identify component compositions of the antimicrobial activity enhancing agents produced in Examples 1 and 2, SDS-PAGE was performed by using NuPAGE 4-12% Bis-Tris Gel (Invitrogen).

Further, staining was performed by using SimplyBlue SafeStain (Invitrogen).

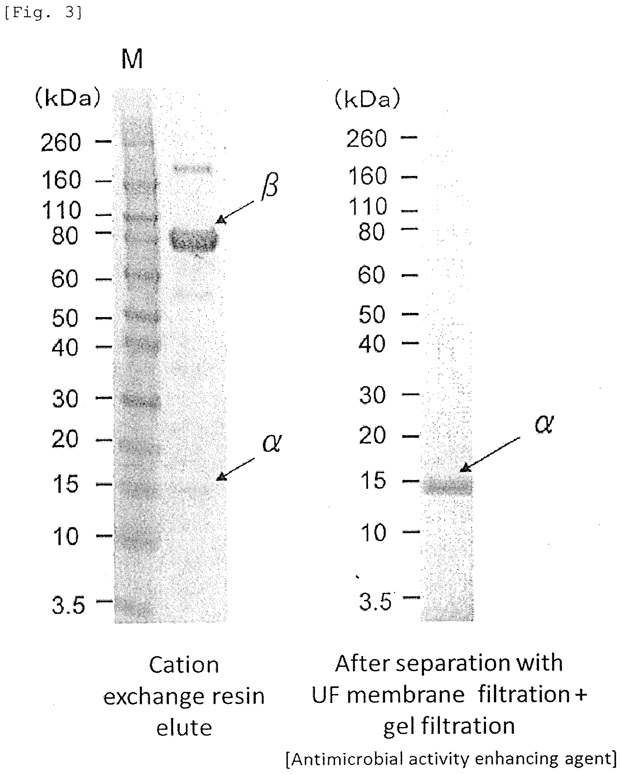

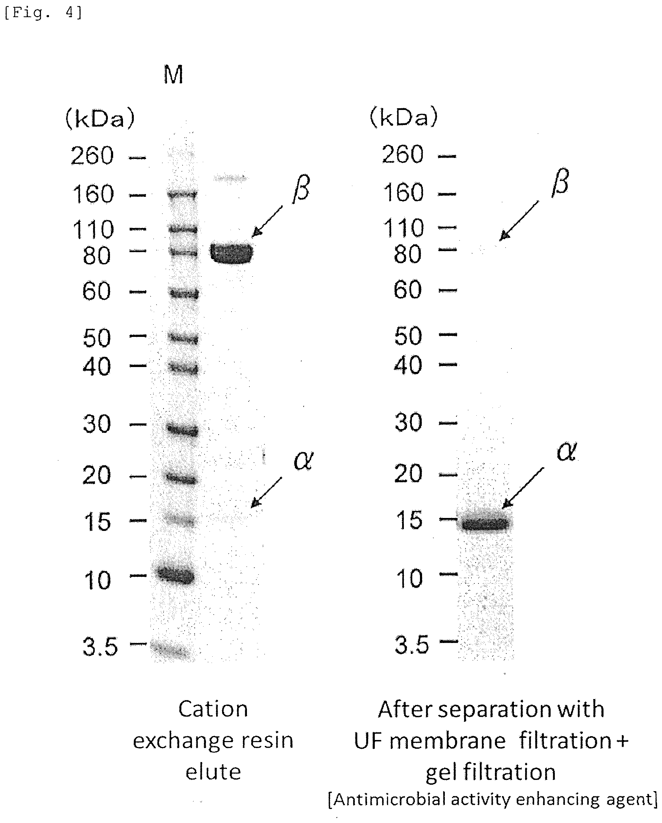

The result of SDS-PAGE of the antimicrobial activity enhancing agent produced in Example 1 is shown in Fig. 3. The result of SDS-PAGE of the antimicrobial activity enhancing agent produced in Example 2 is shown in Fig. 4. In both Figs. 3 and 4, the electrophoretogram of the eluate obtained from the cation exchange resin is shown on the left side (M represents a molecular weight marker), and the electrophoretogram of the antimicrobial activity enhancing agent having undergone the gel filtration treatment is shown on the right side.

As a result, in both of the results of SDS-PAGE, a band $\alpha$ corresponding to 14 kDa was confirmed for the antimicrobial activity enhancing agents of the present invention. In particular, for the antimicrobial activity enhancing agent produced in Example 1, no components other than that of the band $\alpha$ could be confirmed by SDS-PAGE. In the result of SDS-PAGE of the antimicrobial activity enhancing agent produced in Example 2 (Fig. 4), a band $\beta$ slightly stained was detected around 80 kDa.

2) Analysis by mass spectrometry

The band $\alpha$ around 14 kDa fractionated and detected by the SDS-PAGE {Fig. 3 and Fig. 4) was excised from the stained gel for each of the antimicrobial activity enhancing agents of Examples 1 and 2, the portion corresponding to the protein was decolorized, reductively alkylated, digested in the gel by trypsinization, and subjected to mass spectrometry using a MALDI-TOF/MS apparatus (trade name: AutoflexII, BRUKER) to analyze the mass of the peptide.

On the basis of the results of the mass spectrometry, the protein was identified by searching the database of NCBI (National Center for Biotechnology Information, http://www.ncbi.nlm.nih.gov/) using the MASCOT software produced by Matrix Science.

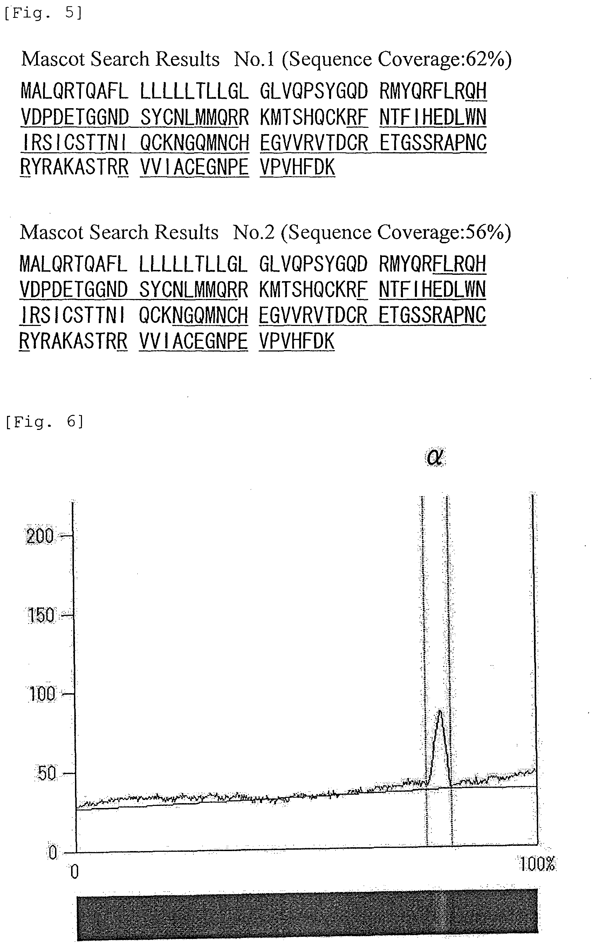

For both of the antimicrobial activity enhancing agents produced in Examples 1 and 2, it was revealed by database search that the band $\alpha$ of 14 kDa separated by SDS-PAGE was a protein derived from bovine ribonuclease-4 (RNase-4), as shown in Fig. 5.

3) Content of active ingredient contained in antimicrobial activity enhancing agent

For each of the antimicrobial activity enhancing agents of Examples 1 and 2, the stained gel having undergone fractionation and detection by SDS-PAGE (shown on the right side in Figs. 3 and 4) was imaged with Densitograph (ATTO), and the band was confirmed by using analysis software. Fig. 6 shows the densitogram for the antimicrobial activity enhancing agent of Example 1. Fig. 7 shows the densitogram for the antimicrobial activity enhancing agent of Example 2.

The densitogram of Fig. 6 was numerically represented as shown in Table 1. As a result, it was revealed that when the antimicrobial activity enhancing agent of the present invention was produced from a raw material derived from milk by the method described in Example 1, ribonuclease-4 as the active ingredient is contained at a ratio of about 100 mass % in the antimicrobial activity enhancing agent. Also on the densitogram, peaks other than the peak α corresponding to ribonuclease-4 were not detected.

In other words, it is meant that by producing ribonuclease-4 from a raw material derived from milk by the method described in Example 1, ribonuclease-4 can be collected with high purity.

[Table 1]

Table 1

| Peak No. | Integrated value | Content (mass %) |
|---|---|---|
| α | 23,165 | 100.0 |

Further, the densitogram of Fig. 7 was numerically represented as shown in Table 2. As a result, it was revealed that when the antimicrobial activity enhancing agent of the present invention was produced from a raw material derived from milk by the method described in Example 2, ribonuclease-4 as the active ingredient identified as the peak α was contained at a ratio of about 96.8 mass % in the antimicrobial activity enhancing agent.

In addition, as for the band β around 80 kDa slightly stained as shown in Fig. 4, the peak β was also detected in the densitogram, and it was confirmed that the peak β was contained in the antimicrobial activity enhancing agent at a ratio of 3.2 mass %.

Further, when the protein of the peak β was identified, it was confirmed that it was a protein derived from lactoferrin. The antimicrobial activity enhancing agent produced by the method described in Example 2 (peak α: 96.8 mass, peak β: 3.2 mass %) per se did not show antimicrobial activity.

[Table 2]

Table 2

| Peak No. | Integrated value | Content (mass %) |
|---|---|---|
| α | 85,527 | 96.8 |
| β | 2,847 | 3.2 |

[Test Example 2]

This test was performed in order to confirm that the antimicrobial activity enhancing agent of the present invention has an enhancing action of the antimicrobial activity of lactoferrin.

(1) Preparation of samples

Bovine lactoferrin (Morinaga Milk Industry) was diluted with deionized water, and used as lactoferrin samples.

The antimicrobial activity enhancing agent produced by the method described in Example 1 (containing ribonuclease-4 at a ratio of 100 mass %) was diluted with deionized water, and used.

Samples 1 to 5 described below were prepared by using the bovine lactoferrin and antimicrobial activity enhancing agent described above.

Sample 1: Bovine lactoferrin (2 mg/mL)
Sample 2: Mixture of bovine lactoferrin (2 mg/mL) and antimicrobial activity enhancing agent (32 pg/mL).

Sample 3: Mixture of bovine lactoferrin (2 mg/mL) and antimicrobial activity enhancing agent (64 pg/mL).
Sample 4: Mixture of bovine lactoferrin (2 mg/mL) and antimicrobial activity enhancing agent (100 μg/mL).
Sample 5: Antimicrobial activity enhancing agent (100 pg/mL)

By using 1% Bacto peptone medium (Becton Dickinson), *Escherichia coli* K-12 strain (NBRC 3301) was inoculated into the medium contained in a polystyrene tube with a 5ml capacity at a density of $10^6$ cells/mL, and precultured in an incubator at 37°C for 6 hours to obtain a cell suspension for the test.

(2) Test method

The cell suspension for the test was added to a 96-well plate at a cell density of $10^6$ cells/mL together with each of the aforementioned samples, and cultured at 37°C for 17 hours.

After the culture for 17 hours, the culture medium containing *Escherichia coli* was serially diluted with 0.85% physiological saline, and plated on an agar medium (Rapid Media RM-SPC, Morinaga Milk Industry), and culture was performed at 37°C for 16 hours. Then, number of the colonies formed on the medium (colony forming unit : CFU) was counted to evaluate the antimicrobial activity of each sample.

In addition, *Escherichia coli* was cultured only with the medium solution as a control, besides the culture using Samples 1 to 5.

(3) Test results

The results of this test are shown in Table 3. The results of the significant test shown in Table 3 satisfy P < 0.001.

Whereas the bacterial count increased to 1.7 x $10^8$ CFU/mL after 17 hours in the control culture, which was performed only with the medium, the bacterial count decreased from the initial bacterial count to 3.6 x $10^5$ CFU/mL in the culture using Sample 1 containing lactoferrin.

In the culture using Sample 3 containing lactoferrin and the antimicrobial activity enhancing agent, the bacterial count became 3.6 x $10^4$ CFU/mL, which was significantly lower than that obtained in the culture using lactoferrin alone.

Furthermore, even with Sample 2 in which the amount of the antimicrobial activity enhancing agent was decreased to 1/2, the bacterial count became 1.8 x $10^4$ CFU/mL, and thus the bacterial count decreased to the same extent as that obtained with Sample 3.

With Sample 4 containing about three-fold amount of the antimicrobial activity enhancing agent compared with Sample 2, significant antimicrobial activity enhancing action was not observed.

On the basis of comparison of the bacterial counts obtained with Samples 1 and 2, it was determined that the antimicrobial activity of Sample 2 was about 19 times or more as compared with the antimicrobial activity of Sample 1. Further, on the basis of comparison of the bacterial counts obtained with Samples 1 and 3, it was determined that the antimicrobial activity of Sample 3 was about 10 times or more as compared with the antimicrobial activity of Sample 1.

With Sample 5 containing the antimicrobial activity enhancing agent alone, the bacterial count became 1.9 x $10^8$ CFU/mL, and it became clear that the antimicrobial activity enhancing agent per se did not have antimicrobial activity. On the basis of the results of this test, it was revealed that the antimicrobial activity enhancing agent of the present invention had an action of enhancing the antimicrobial activity of lactoferrin.

Thus, it was revealed that by preparing a composition comprising an antimicrobial substance such as lactoferrin and the antimicrobial activity enhancing agent of the present invention, a superior composition for antimicrobial use in which the activity of the conventional antimicrobial substance was markedly enhanced could be provided.

[Table 3]

Table 3

| Sample | Bacterial count (CFU/mL) | S.D. | Survival rate based on control (%) | p value by t-Test |
|---|---|---|---|---|
| Control | 166,250,000 | 59,626,816 | - | - |
| Sample 1 | 359,444 | 99,198 | 0.216 | 0.0000004830 (vs. control) |
| Sample 2 | 18,450 | 11,515 | 0.011 | 0.0000000050 (vs. lactoferrin) |

(continued)

| Sample | Bacterial count (CFU/mL) | S.D. | Survival rate based on control (%) | p value by t-Test |
|---|---|---|---|---|
| Sample 3 | 36,271 | 7,528 | 0.022 | 0.0000000010 (vs. lactoferrin) |
| Sample 4 | 371,818 | 94,002 | 0.224 | 0.7783400990 (vs. lactoferrin) |
| Sample 5 | 189,000,000 | 29,240,383 | 113.684 | 0.4480992030 (vs. control) |

[Test Example 3]

This test aimed at confirming that the antimicrobial activity enhancing agent of the present invention enhances the antimicrobial activity of the lactoferrin hydrolysate, Lactoferricin (registered trademark).

(1) Preparation of samples

Bovine Lactoferricin powder obtained by hydrolyzing the bovine lactoferrin (Morinaga Milk Industry) used in Test Example 2 with pepsin, purifying the hydrolysate by high performance liquid chromatography, and lyophilizing the purified product was used.
The bovine Lactoferricin powder was diluted with deionized water and used.
As the antimicrobial activity enhancing agent, the antimicrobial activity enhancing agent obtained in Example 1 was diluted with deionized water and used.
Samples 6 to 8 described below were prepared by using the aforementioned bovine Lactoferricin and the antimicrobial activity enhancing agent.

Sample 6: Bovine Lactoferricin (16 $\mu$g/mL)
Sample 7: Mixture of bovine Lactoferricin (16 pg/mL) and antimicrobial activity enhancing agent (32 $\mu$g/mL)
Sample 8: Antimicrobial activity enhancing agent (32 pg/mL)

(2) Test method

The test was performed by the same test method as that of Test Example 2. Besides the culture using Samples 6 to 8, *Escherichia coli* was cultured only with the medium as a control.

(3) Results

The results of Test Example 3 are shown in Table 4. The bacterial count increased to $1.0 \times 10^8$ CFU/mL after 17 hours in the control, which was cultured only with the medium.
In the culture using Sample 6 containing 16 $\mu$g/mL of the bovine Lactoferricin, the bacterial count decreased to $1.8 \times 10^4$ CFU/mL, but in the culture using Sample 7 further containing the antimicrobial activity enhancing agent, the bacterial count became $2.5 \times 10^2$ CFU/mL or less, and thus marked decrease of the bacterial count was observed.
On the basis of comparison of the bacterial counts obtained with Samples 6 and 7, it was determined that the antimicrobial activity of Sample 7 was 72 times or more as compared with the antimicrobial activity of Sample 6.
With Sample 8 containing the antimicrobial activity enhancing agent alone, the bacterial count became $1.2 \times 10^8$ CFU/mL, and thus antimicrobial activity was not observed at all.
On the basis of the results of this test, it was confirmed that marked increase of the antimicrobial activity was provided by the combination of the bovine lactoferrin hydrolysate and the antimicrobial activity enhancing agent of the present invention.

[Table 4]

Table 4

| Sample | Bacterial count (CFU/mL) | S.D. | Survival rate based on control (%) | p value by t-Test |
|---|---|---|---|---|
| Control | 101,666,667 | 37,771,241 | - | - |
| Sample 6 | 18,167 | 2,962 | 0.018 | 0.0027877104 (vs. control) |
| Sample 7 | 250 | 0 | 0.000 | 0.0004688587 (vs. Lactoferricin) |
| Sample 8 | 122,500,000 | 27,883,687 | 120.492 | 0.3025608131 (vs. control) |

Industrial Applicability

By adding the antimicrobial activity enhancing agent of the present invention to an antimicrobial agent containing lactoferrin or a lactoferrin hydrolysate as an active ingredient, the antimicrobial activity of lactoferrin or the lactoferrin hydrolysate can be markedly improved.

Further, since the antimicrobial activity enhancing agent of the present invention exhibits the effect even when it is mixed only in an extremely small amount with lactoferrin or a lactoferrin hydrolysate, it can be blended with foods, drinks, feeds, drugs, cosmetics, and so forth without affecting composition and properties of the products.

Furthermore, since the active ingredient of the antimicrobial activity enhancing agent of the present invention is ribonuclease-4 obtained from components derived from milk, it scarcely has a risk of adverse reaction, and it can be taken in daily basis with feeling safe.

**Claims**

1. Use of ribonuclease-4 for enhancing the antimicrobial activity of a peptide having the amino acid sequence of SEQ ID NO: 3 derived from lactoferrin, wherein the mass ratio of ribonuclease-4 to the peptide is 1:1 to 4:1.

2. An antimicrobial composition comprising an antimicrobial activity enhancing agent comprising ribonuclease-4 and a peptide having the amino acid sequence of SEQ ID NO: 3 derived from lactoferrin, wherein the mass ratio of ribonuclease-4 to the peptide is 1:1 to 4:1.

3. The antimicrobial composition according to claim 2, wherein the mass ratio of ribonuclease-4 to the peptide is 1:1 to 2.5:1.

4. A drug for antimicrobial use containing the antimicrobial composition according to claim 2 or 3.

5. A food or drink containing the antimicrobial composition according to claim 2 or 3.

6. A feed containing the antimicrobial composition according to claim 2 or 3.


**Patentansprüche**

1. Verwendung von Ribonuclease-4 zur Verbesserung der antimikrobiellen Aktivität eines von Lactoferrin abgeleiteten Peptids mit der Aminosäuresequenz SEQ ID NO: 3, wobei das Massenverhältnis von Ribonuclease-4 zum Peptid 1:1 bis 4:1 beträgt.

2. Eine antimikrobielle Zusammensetzung, umfassend ein Mittel zur Verbesserung antimikrobieller Aktivität, umfassend Ribonuclease-4 und ein von Lactoferrin abgeleitetes Peptid mit der Aminosäuresequenz SEQ ID NO: 3, wobei das Massenverhältnis von Ribonuclease-4 zum Peptid 1:1 bis 4:1 beträgt.

3. Die antimikrobielle Zusammensetzung nach Anspruch 2, wobei das Massenverhältnis von Ribonuclease-4 zum Peptid 1:1 bis 2,5:1 beträgt.

4. Ein Arzneimittel für eine antimikrobielle Verwendung, die die antimikrobielle Zusammensetzung nach Anspruch 2 oder 3 enthält.

5. Ein Nahrungsmittel oder Getränk, welches die antimikrobielle Zusammensetzung nach Anspruch 2 oder 3 enthält.

6. Ein Futtermittel, welches die antimikrobielle Zusammensetzung nach Anspruch 2 oder 3 enthält.


**Revendications**

1. Utilisation de la ribonucléase 4 pour l'amplification de l'activité antimicrobienne d'un peptide ayant la séquence d'acides aminés de SEQ ID NO : 3 dérivé de la lactoferrine, dans laquelle le rapport massique de la ribonucléase 4 sur le peptide est de 1:1 à 4:1.

2. Composition antimicrobienne comprenant un agent amplifiant l'activité antimicrobienne comprenant de la ribonucléase 4 et un peptide ayant la séquence d'acides aminés de SEQ ID NO : 3 dérivé de la lactoferrine, dans laquelle le rapport massique de la ribonucléase 4 sur le peptide est de 1:1 à 4:1.

3. Composition antimicrobienne selon la revendication 2, dans laquelle le rapport massique de la ribonucléase 4 sur le peptide est de 1:1 à 2,5:1.

4. Médicament pour une utilisation antimicrobienne contenant la composition antimicrobienne selon la revendication 2 ou 3.

5. Aliment ou boisson contenant la composition antimicrobienne selon la revendication 2 ou 3.

6. Aliment pour animaux contenant la composition antimicrobienne selon la revendication 2 ou 3.

[Fig. 1]

[Fig. 2]

[Fig. 3]

Cation
exchange resin
elute

After separation with
UF membrane  filtration +
gel filtration

[Antimicrobial activity enhancing agent]

[Fig. 4]

Cation
exchange resin
elute

After separation with
UF membrane  filtration+
gel filtration
[Antimicrobial activity enhancing agent]

[Fig. 5]

Mascot Search Results  No.1 (Sequence Coverage:62%)

MALQRTQAFL LLLLLTLLGL GLVQPSYGQD RMYQRFLRQH
VDPDETGGND SYCNLMMQRR KMTSHQCKRF NTFIHEDLWN
IRSICSTTNI QCKNGQMNCH EGVVRVTDCR ETGSSRAPNC
RYRAKASTRR VVIACEGNPE VPVHFDK

Mascot Search Results  No.2 (Sequence Coverage:56%)

MALQRTQAFL LLLLLTLLGL GLVQPSYGQD RMYQRFLRQH
VDPDETGGND SYCNLMMQRR KMTSHQCKRF NTFIHEDLWN
IRSICSTTNI QCKNGQMNCH EGVVRVTDCR ETGSSRAPNC
RYRAKASTRR VVIACEGNPE VPVHFDK

[Fig. 6]

[Fig. 7]

[Fig. 8]

[Fig. 9]

## REFERENCES CITED IN THE DESCRIPTION

**Patent documents cited in the description**

- JP 11292789 A
- JP 5255109 A
- JP 5320067 A
- JP 6199698 A
- JP 9124504 A
- JP 11092375 A
- US 2008255340 A
- WO 2007142542 A
- JP 3171736 A

**Non-patent literature cited in the description**

- **WELSH, J.K. et al.** *Journal of Pediatrics,* 1979, vol. 94, 1-9
- **NONNECKE, B.J. et al.** *Journal of Dairy Science,* 1984, vol. 67, 606-613
- **BELLAMY, W. et al.** *Biochimica et Biophysica Acta,* 1992, vol. 1121, 130-136
- **BELLAMY, W. et al.** *Journal of Applied Bacteriology,* 1992, vol. 73, 472-479